# EUROPEAN PATENT SPECIFICATION

(11) **EP 1 389 079 B1**
(45) Date of publication and mention of the grant of the patent: **10.12.2008**
(21) Application number: 01965729.5
(22) Date of filing: 06.09.2001
(51) Int. Cl.: A61N 1/378

(54) **APPARATUS FOR CONTACTLESS POWER TRANSFER FOR IMPLANTABLE MEDICAL DEVICE**
GERÄT FÜR DEN KONTAKTLOSEN ENERGIETRANSFER FÜR EINE IMPLANTIERBARE MEDIZINISCHE VORRICHTUNG
APPAREIL PERMETTANT D'EFFECTUER UN TRANSFERT D'ENERGIE SANS CONTACT POUR UN DISPOSITIF MEDICAL IMPLANTABLE

(30) Priority: 23.05.2001 KR 2001028347
(43) Date of publication of application: 18.02.2004
(73) Proprietor: Ahn, Tae Young, Cheongju-si, Chungcheongbuk-do 360-771 (KR); Moon, Chan Il, Seoul 133-777 (KR); Kim, Byoung Joon, Seoul 139-925 (KR)
(72) Inventor: Ahn, Tae Young, Cheongju-si, Chungcheongbuk-do 360-771 (KR); Moon, Chan Il, Seoul 133-777 (KR); Kim, Byoung Joon, Seoul 139-925 (KR)
(74) Representative: Derambure, Christian
(86) International application number: PCT/KR2001/001508
(87) International publication number: WO 2002/094139

(56) References cited:
- JP-A- 1 305 945
- JP-A- 3 151 964
- JP-A- 3 151 980
- KR-A- 870 002 814
- KR-A- 910 016 351
- US-A- 3 357 434
- US-A- 5 487 760
- US-A- 5 948 006
- US-A- 6 032 076
- US-A- 6 092 531

## Description

### TECHNICAL FIELD

The present invention relates to an implantable medical device. In particular, the invention relates to an apparatus for transcutaneously transmitting electrical power to a medical device implanted in a living body.

### BACKGROUND ART

In the field of medical implantation, a reduction in the size and weight of implanted devices is the major challenge for many reasons. The size of an implanted device directly affects the comfort of the patient. Particularly, if an implant is large it will require that much large opening in the living body either to insert or remove it, possibly causing an excessive bleeding and increasing vulnerability to infection during the implantation.

A battery occupies 50 to 80 % of volume in most of implanted medical devices. But, batteries have a limited life span so that they have to be replaced periodically. The replacement also requires a surgical operation to make an opening in the body, which is very inconvenient to and can be dangerous for some patients.

To solve the problem, researchers have been studying how to supply power to implanted medical devices non-surgically. A solution was a transcutaneous power transmission (non-contact type power transmission). However, known charging devices have failed to satisfy some of the basic requirements such as miniaturization and stability under a harsh environment.

A prior art charger for implanted medical device is disclosed in U. S. patent No. 4,143,661. It discloses implanting a very large coil in a human body so as to surround a leg or the waist to use it as the secondary coil. Implanting such a large coil adversely affects the patient's condition. In addition, a large coil inserted into a human body could cause damages to the body.

Another prior art charger is disclosed in U. S. patent No. 5,358,514. The charger disclosed therein includes a secondary transformer, a battery and other supplemental circuitry. For magnetic flux supplied from outside of a human body to reach the charger, the charger cannot be enclosed in a metal case, which imposes restrictions on the design of the implanted device. Since ferromagnetic core surrounded by a coil is used as a component of a secondary transformer, it is bulky and vulnerable to impact from outside.

Document US 6,092,531 discloses a motor that energizes either one or more moving permanent magnets, or one or more moving element to vary the magnetic field and to induce power in an implanted receiver coil within a patient's body.

Document US 5,948,006 discloses a transcutaneous transmission patch made of a flexible material capable of transcutaneously transmitting power and/or data through an external coil to a receiving coil housed in an implanted device.

US-A-5 487 760 refers to powering transcutaneously an implanted circuit by RF-transmitted energy. A printed circuit coil antenna is disclosed.

### DISCLOSURE OF THE INVENTION

It is, therefore, an objective of the present invention to provide a transcutaneous power transmission apparatus for use in an implantable medical device.

In accordance with one aspect of the present invention, there is disclosed an apparatus for providing power to an implantable medical device in a living body, comprising a means for receiving power in the form of magnetic flux from an external power source and providing the received power to said implantable medical device, wherein said means includes a flexible coil.

In accordance with another aspect of the present invention, there is disclosed an apparatus for providing power to an implantable medical device, comprising: a first circuit for transmitting power in the form of magnetic flux; and a second circuit for receiving said power from said first circuit and for providing the received power to said implantable medical device, wherein said first and second circuits are not physically coupled and said second circuit includes a flexible coil.

In accordance with another aspect of the present invention, there is provided an implatable medical device, comprising:
a first circuit for receiving power in the form of magnetic flux and providing the received power; and
an electrical device using the power provided from said first circuit, wherein said first circuit comprises a flexible coil.

In accordance with yet another aspect of the present invention, there is provided an apparatus for providing power to an implantable medical device, comprising a first circuit for receiving power in the form of magnetic flux and providing the received power to said implanted medical device, wherein said first circuit includes a substantially flat coil.

### BRIEF DESCRIPTION OF THE DRAWINGS

The above and other objects and features of the present invention will become apparent from the following description of preferred embodiments given in conjunction with the accompanying drawings, in which:
Fig. 1 shows a schematic diagram of a medical device including a flat type coil in accordance with the present invention;
Figs. 2A and 2B are diagrams illustrating front and sectional views of a primary coil in accordance with an embodiment of the present invention;
Fig. 3A through Fig. 3D are diagrams illustrating front and sectional views of a secondary coil in accordance with an embodiment of the present invention;
Fig. 4A, Fig. 4B and Fig. 4C are diagrams illustrating embodiments of transformers in which primary and secondary coils are coupled together;
Fig. 5A is a schematic diagram of an experimental circuit for confirming an operation of a transformer used in the present invention;
Fig. 5B shows voltage waveforms obtained from a test for confirming an operation of a transformer used in the present invention;
Fig 6. is a functional block diagram of one embodiment of a charging system in accordance with the present invention;
Fig 7. is a functional block diagram of another embodiment of a charging system in accordance with the present invention;
Fig 8. is a functional block diagram of yet another embodiment of a charging system in accordance with the present invention;
Fig. 9 is a diagram illustrating a first embodiment of a supplementary tool for using the charging system according to the present invention; and
Fig. 10 is a diagram illustrating a second embodiment of a supplementary tool for using the charging system according to the present invention.

### BEST MODE FOR CARRYING OUT THE INVENTION

An embodiment of the present invention will now be described in details with reference to the accompanying drawings.

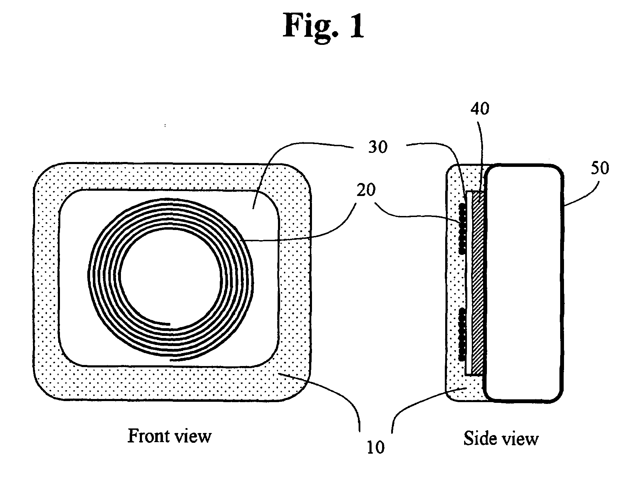

A top view of an implantable medical device is shown in Fig.1, having a concentrically-wound coil ("flat coil") that is inductively coupled to an external coil (not shown in Fig. 1). The two coils, acting as primary and secondary windings, form a transformer such that power from an external source connected to the external coil can be inductively transferred to a battery coupled to the flat coil.

In accordance with the present invention, the flat coil 20 is made small so that it can be attached onto an exterior surface of often small implantable medical device. Known methods for forming a flat coil on a printed circuit board, preferably a flexible PCB, can be utilized. However, the present invention is not confined to these methods but one may utilize any other method for arranging a substantially flat coil onto a surface of an implanted medical device.

Flexible PCBs are widely used in small devices such as wristwatches and cameras. Flexible PCBs are made, for example, by pressing plastic material at both sides of metal wire (s).

There are various methods for making flat type coils by mounting a coil on a flexible PCB. One of such methods is the patterning of a coil on a substrate. This method comprises printing a coil and other circuit components and thereafter removing remaining parts by chemical processes. In this method, it is possible to mount related control circuits on the same circuit board concurrently with patterning the coil.

In addition to the above patterning method, it is possible to concentrically wind a copper wire and attach the coil onto a substrate. Although the coil depicted in Fig. 1 is completely flat, it may not be so if a surface of an implantable device, onto which the coil is supposed to be attached or formed, is curved. In other words, the "flat" coil actually can follow the contour of a flexible circuit board. This flexibility of the flat coil provides an advantage because the shape of a medical device does not have to be restricted just in order to accommodate the coil.

The gauge, number of turns, length of a coil depend on factors such as desired power transmission, distance from the primary coil outside the living body and battery charging time.

In one embodiment of the present invention, the coil and a PCB onto which the coil is formed are covered with a medically-safe material such silicon or latex 10 as shown in Fig. 1 in order to prevent corrosion the coil and PCB circuits and also prevent a possible release of foreign materials from the device inside a living body.

To prevent magnetic flux generated from nearby electronic devices from affecting the medical device, a magnetic shield layer 40 may be placed between the coil-formed flexible substrate and the main body of an implantable medical device. The most common method for shielding magnetic flux is to surround the device with ferromagnetic materials or compounds.

In the present invention, ferrite compounds in liquid phase, film shape, or solid phase can be utilized as the shield layer 40. The ferrite compounds in liquid phase include a shielding paint that is a mixture of paint and ferrite powder for absorbing electromagnetic flux produced by, for example, computer monitors, and magnetic fluid for use with speakers. Examples of such liquid ferrite compound include SMF series products that are produced by Samhwa Electrics (See www.samhwa.co.kr/english/products/98.asp). Film type ferrite material includes ferrite polymer compound film supplied by Siemens of Germany (See www.epcos.de/inf/90/ap/e0001000.htm). Because materials in solid state are vulnerable to impact and difficult to handle, it is preferable to utilize materials in liquid state or film shape depending on the shapes of the medical device or the part in a human body where the device is inserted.

In another embodiment of the present invention, a coil is patterned on the housing of an implanted medical device, which simplifies the overall manufacturing process. In this case, a shield layer is constructed by coating a ferrite compound on the device, followed by printing a coil pattern. Then, the entire outer surface of the apparatus is coated with a silicon material.

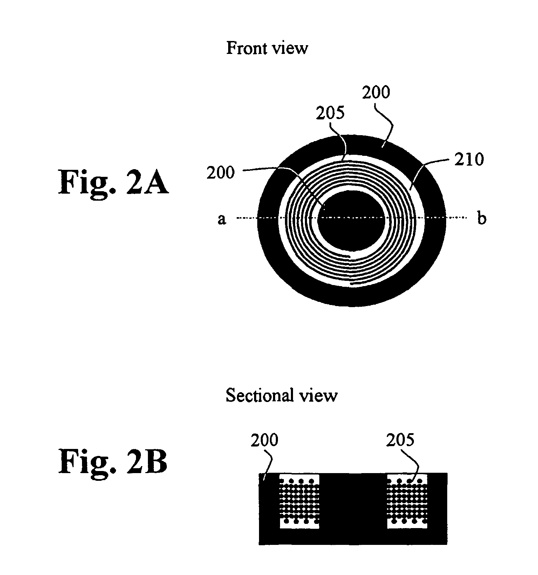

Fig. 2A and Fig. 2B are diagrams illustrating, respectively, front and cross-sectional views of the primary coil according to one embodiment of the present invention. It is preferable to construct the shape of the primary coil so as to efficiently transmit power to the secondary coil positioned in a human body. As one example, a primary coil of Fig. 2 is placed concentrically inside a hollow cylinder of magnetic having a center column in the coaxial direction. In other words, conductive wire is wound around the column inside the hollow cylinder. When a current is supplied to the coil 205, magnetic flux is produced in the coaxial direction. Thus, power transmission efficiency is enhanced by placing the flat coil inside the living body and the primary coil assembly such that they are in parallel.

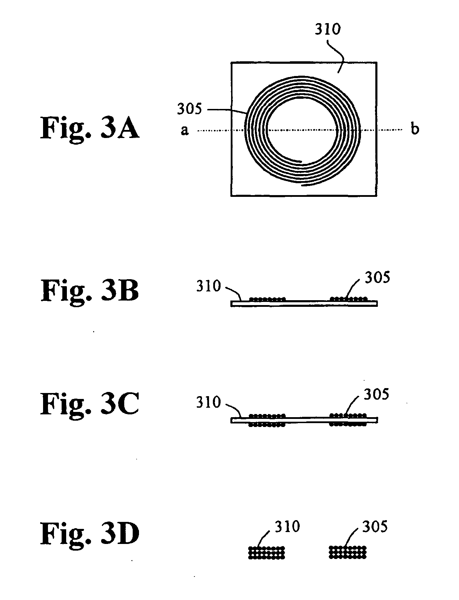

Fig. 3A through Fig. 3D are diagrams illustrating front and cross-sectional views of an exemplary secondary coil. The coil may be printed onto one or both sides of the substrate, as shown in Figs. 3B and 3C. As described above, the substrate illustrated in Fig.3B and Fig.3C may be flexible. Fig. 3D depicts an embodiment that does not include a substrate. In place of the substrate, the separately-prepared coil is directly attached onto the housing of a medical device.

In Figs. 3, the secondary coil is depicted as a concentric winding. But the present invention is not limited to this shape. Other designs include meander and mesh shapes. What is important is that the overall shape of the secondary coil is determined depending on the shape of its target medical device and on a desired efficiency of transformer.

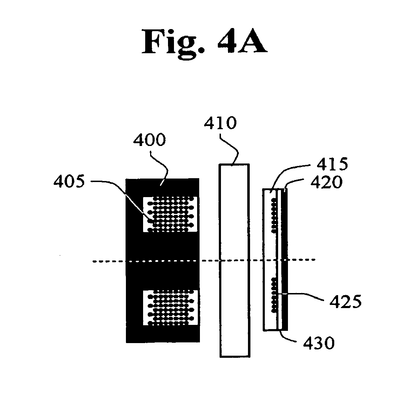

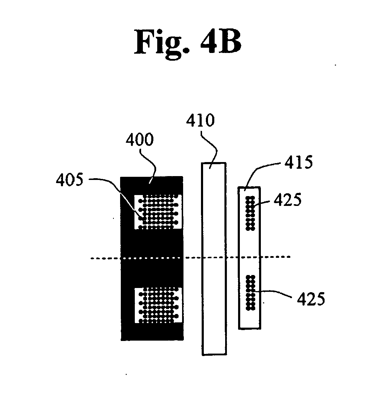

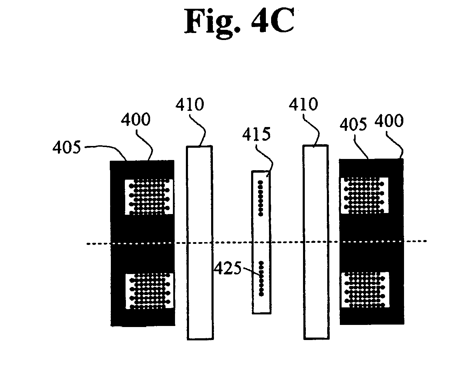

Figs. 4A, 4B, and 4C illustrate embodiments where the primary and secondary coils are inductively coupled in various arrangements. The primary coil 405 is disposed outside of a human body and the secondary coil 425 is disposed inside of the human body 410

Fig. 4A illustrates an embodiment in which the secondary coil is attached to (or printed on) the substrate 430 and a shielding film 420 is disposed under the substrate. In Fig. 4B, the secondary coil 425 is coated with a protective material such as silicon. In Fig. 4C, two primary coils are disposed off both sides of the human body to increase power transmission.

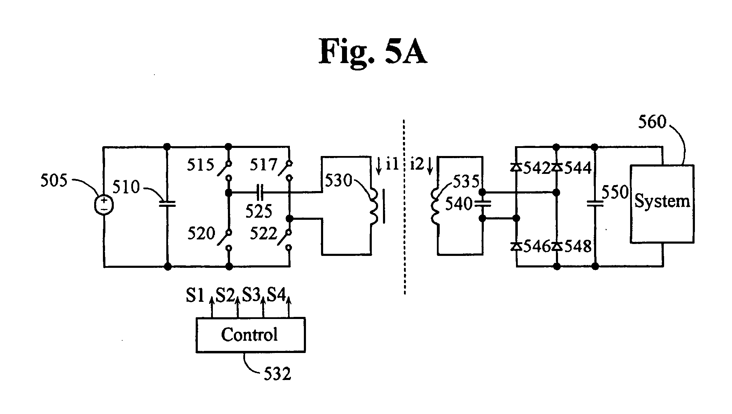

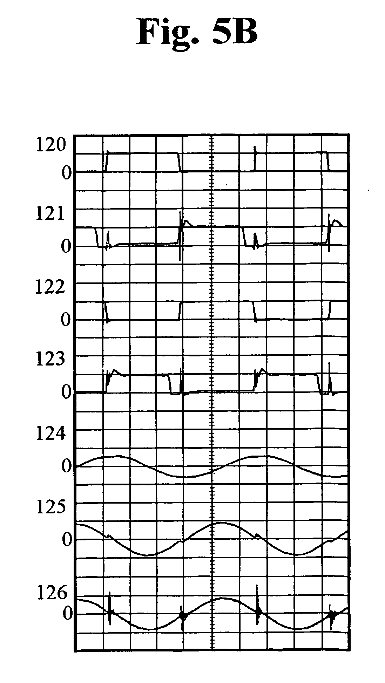

Fig. 5A depicts a circuit modeling of power transmission according to the present invention. In operation, a current is provided from an external power source 505, and switches 515, 517, 520, and 522 are controlled by control signals s1, s2, s3, and s4 (See waveform 120 to 123) produced in a control means 532. When AC current i1 flows in the primary coil 530 by the operation of the switches and a capacitor 525, current i2 is induced in the inductively-coupled secondary coil 535, having substantially the same waveform of current i1. The AC current i2 is rectified to a direct current by diodes 542, 544, 546 and 548. The resultant direct current is provided to charge a rechargeable battery of the medical device 560. Fig. 5B depicts waveforms of the control signals and the currents in the primary and secondary windings.

Any known circuits for charging a rechargeable battery may be used. Examples of such circuits are MAX745, MAX1679, MAX1736, MAX1879 provided by MAXIM and LTC1732-4/LTC1732-4.2 and LT1571 series provided by Linear technology.
(see
http://dbserv.maxim-ic.com/quick view2.cfm?qv pk=2544,
http://dbserv.maxim-ic.com/quick view2.cfm?qv pk=2094,
http://dbserv.maxim-ic.com/quick view2.cfm?qv pk=2219,
http://dbserv.maxim-ic.com/quick view2.cfm?qv pk=1661,
http://www.linear-tech.com/prod/datasheet.html?datasheet=606,
http://www.linear.com/prod/datasheet?datasheet=575&produc t family=power) .

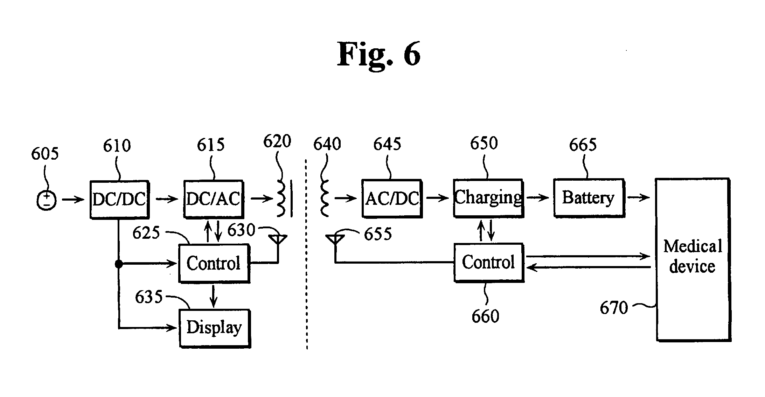

Fig. 6 depicts a functional block diagram of one embodiment of a charging system in accordance with the present invention. The DC power from an external power source 605 is fed first to a DC/DC converter 610 in order to stabilize the voltage. The stabilized DC power is provided to a DC/AC converter 615. The AC current from the DC/AC converter is applied to the primary coil 620. Generally, a high frequency AC voltage is supplied to the primary coil 620 in order to enhance efficiency of non-contact type power transmissions as well as to make the transformer smaller and lighter. Therefore, the DC/AC converter 615 used in the embodiment should be chosen such that it produces an AC current of with a frequency range of several hundreds kHz.

At the secondary side in a human body, power transmitted from the primary coil 620 is converted to direct current by a rectifier (an AC/DC converter 645) and then made constant voltage by a charging means 650 and a control means 660 so as to charge a battery 665. It is preferable to use a small and stable battery in the medical device. Lithium-ion and lithium-polymer batteries are examples of small and thin batteries. Although the lithium-ion battery is more efficient, the lithium-polymer battery is preferable because it is more stable.

In the embodiment depicted in Fig. 6, the control means 625, 660 can communicate wirelessly with each other. Antennas 630, 655 are coupled to the control means to transmit and receive radio frequency signals. For example, data related to the charging operation (for example, the battery voltage) may be transferred from the secondary side to the primary side. This data may be used by the primary control means 625 to control the DC/AC converter 615 and can be displayed on a display 635 for an operator to monitor the charging process.

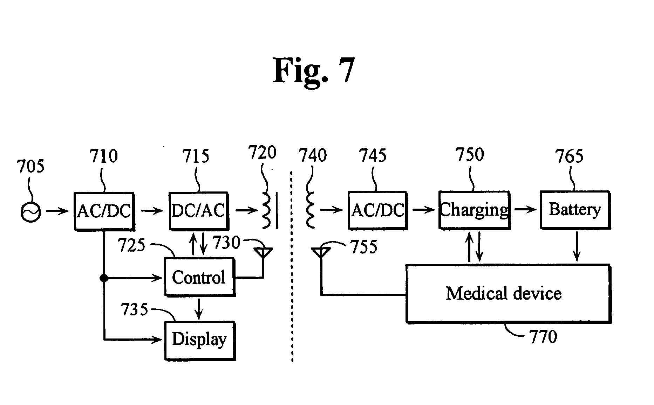

Fig. 7 is a functional block diagram of another embodiment of the charging device in accordance with the present invention. The embodiment depicted in Fig.7 is similar to the embodiment of Fig. 6 except that the controller in the secondary side is integrated with a medical device 770; and an AC source 705 is utilized as a power source at the primary side. As explained above with reference to Fig.6, high frequency current is preferably used for contactless power transfer. For this, the standard AC current of 60 Hz is first converted to a DC current by an AC/DC converter 710 before converted back to an AC current of a much higher frequency by DC/AC converter 715. The resultant AC current is fed to the primary coil 720.

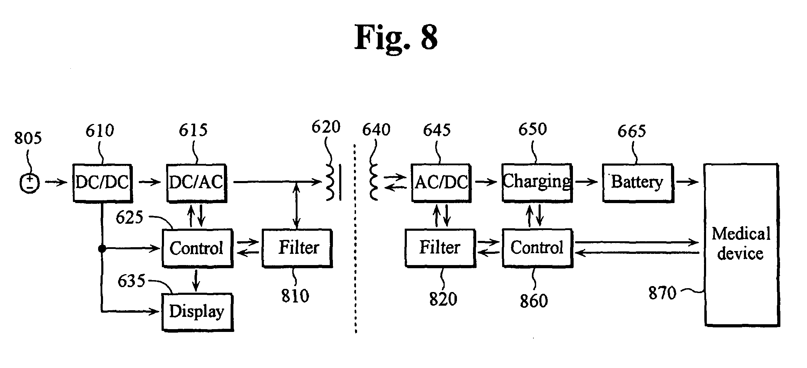

Fig. 8 is a functional block diagram of another embodiment of a charging system in accordance with the present invention. The embodiment of Fig. 8 is similar to those in Figs. 6 and 7 except that filters are provided in primary and secondary sides instead of the antennas. The filters 810, 820 superpose and retrieves a data signal on and from the power signal that is inductively transmitted form the primary winding to the secondary winding. Known methods of using power line for data communication can be utilized for embedding data in the power signal. For example, an information signal in a different bandwidth is combined with the power signal, transmitted to the other side and recovered by separating the information signal form the power signal.

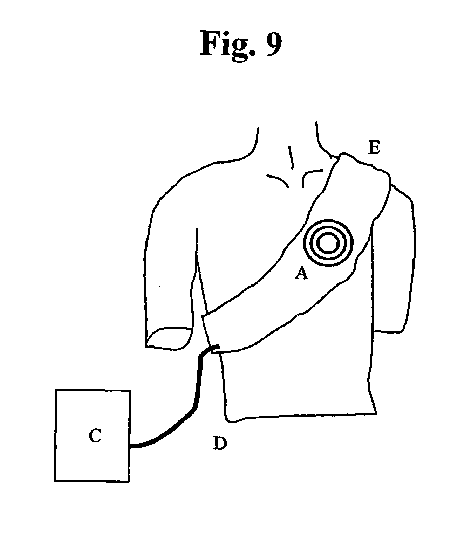

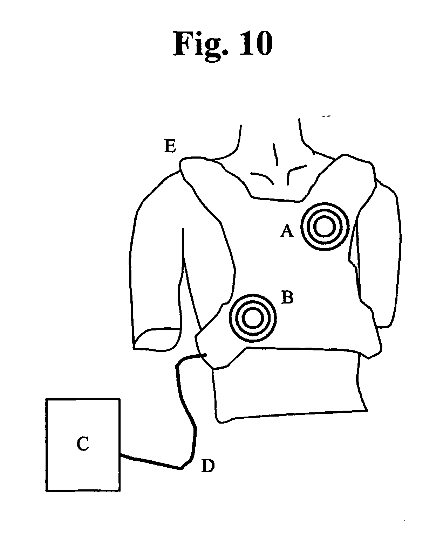

Fig. 9 is a diagram illustrating a first embodiment of a supplementary tool that a human user can wear according to the present invention. It is necessary to place the primary coil on the human body such that it is as close as possible to the secondary coil for efficient power transmission. For this purpose, a belt or vest with the primary winding assembly built in is provided as shown in Figs. 9 and 10. The primary coil can be disposed at various points on the belt/vest. Power is transferred from an external power source C to the primary coil via a power cable D.

The transcutaneous power transmission apparatus according to the present invention can be utilized for various implantable medical devices that requires electrical power, such as an artificial heart, a pacemaker, an implantable cardiverter defibrillator, a neurostimulator, a GI stimulator, an implantable drug infusion pump, a bone growth stimulation device and many other device.

In accordance with the non-contact type power transmission apparatus of the present invention, electric power can be transmitted to the medical device repeatedly without having to take the implanted medical device out of a human body by making an incision in the body. Thus, it is possible to prevent various bad effects accompanied by the surgery. Further, the whole apparatus can be made lighter and smaller since the size of a battery can be reduced, thereby reducing the side effects caused by the implantation.

In addition, it is possible to substantially extend the life span of a medical device because of the use of rechargeable batteries. In addition, since the secondary coil can be formed in a variety of shapes, it is easy to design medical devices that accommodate the inside of a living body. Especially, the medical device can be made very thin.

While the invention has been described with reference to its preferred embodiments, it will be apparent to those skilled in the art that variations and modifications are possible without deviating from the broad principles and teachings of the present invention which should be limited solely by the scope of the claims appended hereto.

## Claims

1. Apparatus for providing power to an implantable medical device in a living body, comprising a means for receiving power from an external power source and providing the received power to the implantable medical device, including:
- a battery (665, 765) intended to be inside the implantable medical device (670, 770, 870);
- a primary coil (205, 405) for transmitting the power in the form of magnetic flux when a current is supplied from the external power source (505, 605, 705, 805) ,
- a secondary coil (20, 305, 425) for receiving the power in the form of the magnetic flux from the primary coil (205, 405) and charging the battery (665, 765), wherein the secondary coil is intended to be disposed inside the living body in parallel to the primary coil (205, 405) and inductively coupled to the primary coil to form a transformer,
**characterized in that** the primary coil is disposed concentrically inside a hollow cylinder (200, 400) and the magnetic flux is produced in the coaxial direction, the hollow cylinder (200, 400) having a center column in the coaxial direction, wherein the hollow cylinder (200, 400) is thus disposed outside uf the living body ; and
the secondary coil (20, 305, 425) is a flat type coil formed on a flexible printed circuit board (30, 310) and attached onto an exterior surface of the implantable medical device.

2. Apparatus according to claim1, wherein at least a portion of outer surface of the implantable medical device is covered by magnetic flux shielding material.

3. Apparatus according to claim 1, wherein the magnetic flux shielding material includes ferromagnetic materials.

4. Apparatus according to claim 1, wherein at least part of the secondary coil (20, 305, 405) is covered by material (10) for protecting said coil.

## Patentansprüche

1. Gerät für die Lieferung von Strom an eine einpflanzbare medizinische Vorrichtung in einem lebenden Körper, das ein Mittel für den Empfang des Stroms von einer äußeren Stromquelle umfaßt und den erhaltenen Strom an die einpflanzbare medizinische Vorrichtung übergibt, die folgende Teile umfaßt:
- Ein Akku (665, 765), der im Innern der einpflanzbaren medizinischen Vorrichtung (670, 770, 870) sein soll;
- Eine Primärspule (205, 405) für die Übertragung des Stroms in Form eines magnetischen Flusses, wenn ein Strom von der äußeren Stromquelle (505, 605, 705, 805) geliefert wird;
- Eine Sekundärspule (20, 305, 425) für den Empfang des Stroms in Form des magnetischen Flusses von der Primärspule (205, 405) und das Laden des Akkus (665, 765), wobei die Sekundärspule im Innern des lebenden Körpers parallel zur Primärspule (205, 405) angeordnet und induktiv mit der Primärspule gekoppelt werden soll, um einen Wandler zu bilden,
**dadurch gekennzeichnet, daß** die Primärspule konzentrisch im Innern eines hohlen Zylinders (200, 400) angeordnet ist und der magnetische Fluß in der koaxialen Richtung erzeugt wird, wobei der hohle Zylinder (200, 400) eine Mittelsäule in der koaxialen Richtung hat, wobei der hohle Zylinder (200, 400) somit außerhalb des lebenden Körpers angeordnet wird, und daß
die Sekundärspule (20, 305, 425) von der Art einer Flachspule ist, die auf einer flexiblen gedruckten Schaltung (30, 310) gebildet wird und auf einer äußeren Fläche der einpflanzbaren medizinischen Vorrichtung befestigt ist.

2. Gerät nach Anspruch 1, **dadurch gekennzeichnet, daß** zumindest ein Teil der äußeren Fläche der einpflanzbaren medizinischen Vorrichtung von Schirmmaterial gegen magnetische Flüsse abgedeckt wird;

3. Gerät nach Anspruch 1, **dadurch gekennzeichnet, daß** das Schirmmaterial gegen magnetische Flüsse ferromagnetisches Material umfaßt;

4. Gerät nach Anspruch 1, **dadurch gekennzeichnet, daß** zumindest ein Teil der Sekundärspule (20, 305, 405) von Material (10) zum Schutz der besagten Spule abgedeckt ist.

## Revendications

1. Appareil permettant de fournir du courant à un dispositif médical implantable dans un corps vivant, comprenant un moyen de réception du courant provenant d'une source de courant extérieure et fournissant le courant reçu au dispositif médical implantable, comprenant :
- une batterie (665, 765) destinée à se trouver à l'intérieur du dispositif médical implantable (670, 770, 870) ;
- une bobine principale (205, 405) permettant de transmettre le courant sous la forme d'un flux magnétique lorsqu'un courant est fourni à partir de la source de courant extérieure (505, 605, 705, 805),
- une bobine secondaire (20, 305, 425) permettant de recevoir le courant sous la forme du flux magnétique provenant de la bobine principale (205, 405) et de charger la batterie (665, 765), dans lequel la bobine secondaire est destinée à être disposée à l'intérieur du corps vivant, parallèlement à la bobine principale (205, 405) et couplée, de manière inductive, à la bobine principale pour former un transformateur,
**caractérisé en ce que** la bobine principale est disposée, de manière concentrique, à l'intérieur d'un cylindre creux (200, 400) et le flux magnétique est produit dans la direction coaxiale, le cylindre creux (200, 400) possédant une colonne centrale dans la direction coaxiale, dans lequel le cylindre creux (200, 400) est ainsi disposé à l'extérieur du corps vivant ; et
la bobine secondaire (20, 305, 425) est une bobine du type plate, formée sur une carte à circuit imprimé souple (30, 310) et fixée sur une surface extérieure du dispositif médical implantable.

2. Appareil selon la revendication 1, dans lequel au moins une portion de la surface extérieure du dispositif médical implantable est revêtue du matériau de protection du flux magnétique.

3. Appareil selon la revendication 1, dans lequel le matériau de protection du flux magnétique inclut des matériaux ferromagnétiques.

4. Appareil selon la revendication 1, dans lequel au moins une partie de la bobine secondaire (20, 305, 405) est revêtue d'un matériau (10) destiné à protéger ladite bobine.
